(19) Europäisches Patentamt
European Patent Office
Office européen des brevets

(11) Veröffentlichungsnummer: **0 210 532 B1**

(12) # EUROPÄISCHE PATENTSCHRIFT

(45) Veröffentlichungstag der Patentschrift: **11.12.91**

(51) Int. Cl.$^5$: **C12N 9/02**, //C12N9/96

(21) Anmeldenummer: **86109675.8**

(22) Anmeldetag: **15.07.86**

(54) **Verfahren zur Reinigung und gegebenenfalls Gewinnung von Formiat-Dehydrogenase (FDH) aus Candida boidinii und FDH-haltiges Produkt.**

(30) Priorität: **30.07.85 DE 3527268**

(43) Veröffentlichungstag der Anmeldung:
**04.02.87 Patentblatt 87/06**

(45) Bekanntmachung des Hinweises auf die Patenterteilung:
**11.12.91 Patentblatt 91/50**

(84) Benannte Vertragsstaaten:
**AT CH DE FR GB IT LI NL SE**

(56) Entgegenhaltungen:
**WO-A-84/04309**
**FR-A- 2 252 351**

**AFFINITY CHROMOTOGRAPHY AND RELATED TECHNIQUES, 1982, Seiten 491-501, Elsevier Scientific Publishing Co., Amsterdam, NL; K.H. KRONER et al.: "Affinity partition studied with glucose-6-phosphate dehyderogenase in aqueous two-phase systems in response to triazine dyes"**

(73) Patentinhaber: **Gesellschaft für Biotechnologische Forschung mbH (GBF)**
**Mascheroder Weg 1**
**W-3300 Braunschweig-Stöckheim(DE)**

(72) Erfinder: **Cordes, Arno, Dr. Dipl.-Ing.**
**Rottenkamp 15**
**W-3320 Salzgitter 1(DE)**
Erfinder: **Kula, Maria-Regina, Dr. Dipl.-Chem.**
**Selgenbusch 12**
**W-5162-Niederzier-Hambach(DE)**
Erfinder: **Kroner, Karl-Heinz**
**Schneekoppeweg 30**
**W-3340 Wolfenbüttel(DE)**
Erfinder: **Stach, Wolfgang**
**Clausbruchstrasse 4**
**W-3320 Salzgitter 41(DE)**

(74) Vertreter: **Boeters, Hans Dietrich, Dr. et al**
**Boeters & Bauer Bereiteranger 15**
**W-8000 München 90(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

JOURNAL OF CHROMOTOGRAPHY, Band 259, 1983, Seiten 97-105, Elsevier Scientific Publishing Co., Amsterdam, NL; G. KOPPERSCHLÄGER et al.: "Application of affinity partitioning in an aqueous two-phase system to the investigation of triazine dye-enzyme interactions"

J. CHEM. TECH. BIOTECHNOL., Band 32, 1982, Seiten 130-137, Society of Chemical Industry; K.H. KRONER et al.: "Scale-up of formate dehydrogenase by partition"

BIOCHEM. SOC. TRANS., Band 7, Nr. 1, 1979, Seiten 1-5; M.-R. KULA et al.: "Large-scale isolation of enzymes"

JOURNAL OF CHROMATOGRAPHY, Band 376, 1986, Seiten 375-384, Elsevier Science Publishers B.V.; A. CORDES et al.: "Process design for large-scale purification of formate dehydrogenase from Candida boidinii by affinity partition"

## Beschreibung

Formiat-Dehydrogenase (FDH) liegt in Candida boidinii intrazellulär vor. Bisher geht man zur Gewinnung von FDH nach zwei verschiedenen Verfahren vor. So wird in Eur. J. Biochem., 62 (1976), 151 bis 160, ein Verfahren beschrieben, bei dem Candida boidinii kultiviert wird, die geernteten Zellen eingefroren, in einem wässerigen phosphathaltigen Medium stehengelassen und danach mechanisch zerkleinert werden, die Zelltrümmer abzentrifugiert werden, Restprotein mit Streptomycinsulfat ausgefällt und der Niederschlag abzentrifugiert wird, wonach die überstehende Lösung einer Ionenaustauschchromatographie an DEAE-Cellulose zur Gewinnung des Enzyms unterworfen wird. Aus J. Chem. Tech. Biotechnol., 32 (1982) 130 bis 137, ist ferner ein Verfahren bekannt, bei dem Candida boidinii kultiviert wird, die geernteten Zellen mechanisch zerkleinert werden, die erhaltene Zellsuspension einer Hitzedenaturierung unterworfen wird und die resultierende Suspension vier aufeinanderfolgenden Phasenverteilungsstufen unterworfen wird, wobei wässerige 2-Phasensysteme mit Polyethylenglykol und Kaliumphosphat als Phasenbildnern angewendet werden.

Außerdem sind eine Reihe von Arbeiten über die Affinitätsverteilung nicht-intrazellulärer Enzyme in Phasensystemen mit einem Gehalt an Triazin-Farbstoffen bekanntgeworden, die an Polyethylenglykol gebunden sind; vgl. beispielsweise Kroner et al. in Gribnau et al., Affinity chromatography and related techniques, Seiten 491 bis 501, Elsevier, Amsterdam 1982; Cordes et al. in Publ. 3rd Eur. Congr. Biotechnology, Munich 1984, 8d. III, Seiten 557 bis 564; Eur. J. Biochem., 131 (1983) 589 bis 594; J. Chromatography, 259 (1983) 97 bis 105; Analytical Biochemistry, 136 (1984) 264 bis 271; und J. Chromatography, 298 (1984) 483 bis 493. Schließlich wird in Analytical Biochemistry, 124 (1982) 117 bis 124, ein Verfahren beschrieben, bei dem von Bäckerhefe ausgegangen, Phosphofruktokinase als nicht-interzelluläres Enzym mit Polyethylenglykol gefällt und danach in einem wässerigen 2-Phasensystem einer Phasenverteilung unterworfen wird, wobei das System Dextran, Polyethylenglykol und Cibacron-blue-S3G-A® als Triazin-Farbstoff umfaßt, der an Polyethylenglykol gebunden ist.

Bei dem bekannten Verfahren zur Reinigung und gegebenenfalls Gewinnung von FDH besteht jedoch ein Bedürfnis, Reinigungsschritte einzusparen.

Schließlich beschreiben Kula et al. in Biochem. Soc. Trans., 1979 (7) 1 bis 5, ein Verfahren zur Reinigung von Formiat-Dehydrogenase (FDH) aus Candida boidinii, bei dem man (a) das Enzym enthaltende Zellen aufschließt, (b) eine Phasenverteilung (Affinitätsextraktion) mit einem wässerigen 2-Phasensystem durchführt, in dessen einer Phase ein Triazinfarbstoff (Ligand) angereichert ist, nämlich Cibacron-Blue-3G-A $^{R}$, der an ein inertes wasserlösliches Polymeres gebunden ist, und (c) das Enzym gegebenenfalls von der Phase abtrennt, in der es angereichert ist. Dieses bekannte Verfahren führt jedoch zu keiner befriedigenden Enzymausbeute.

Erfindungsgemäß wird dazu ein Verfahren zur Reinigung und gegebenenfalls Gewinnung von Formiat-Dehydrogenase (FDH) aus Candida boidinii vorgeschlagen, bei dem man

(a) das Enzym enthaltende Zellen aufschließt,

(b) eine Phasenverteilung (Affinitätsextraktion) mit einem wässerigen 2-Phasensystem durchführt, in dessen einer Phase ein Triazinfarbstoff (Ligand) angereichert ist, der an ein inertes weasserlösliches Polymeres gebunden ist, und

(c) das Enzym gegebenenfalls von der enzymreichen Phase abtrennt und gegebenenfalls gewinnt, wobei dieses Verfahren dadurch gekennzeichnet ist, daß man bei der Stufe (b)

- gegebenfalls ohne vorherige Abtrennung des Zellmaterials und
- gegebenenfalls ohne vorherige Fremdproteinabtrennung
- die Phasenverteilung mit einem wässerigen 2-Phasensystem durchführt, in dessen einer Phase Procion-red-HE3b® (Ligand) angereichert ist, der an ein inertes wasserlösliches Polymeres gebunden ist,

Erfindungsgemäß wurde also überraschenderweise festgestellt, daß man FDH ohne vorherige Abtrennung von Zellmaterial und Fremdprotein reinigen kann, wenn man FDH einer Phasenverteilung in einem wässerigen 2-Phasensystem unterwirft, das einen Triazin-Farbstoff nämlich Procion-red-HE3b®, umfaßt, der an ein inertes wasserlösliches Polymeres gebunden ist.

Bei einer Ausführungsform des erfindungsgemäßen Verfahrens werden die Candida-boidinii-Zellen nach vorhergehendem Einfrieren in einem wässerigen phosphathaltigen Medium suspendiert und in der Suspension bis zu einem Enzymaustritt von maximal etwa 90, 85 oder 75 % belassen, wonach die erhaltene Suspension der Phasenverteilung unterworfen wird. Das phosphathaltige Medium kann Kaliumphosphat und/oder Kaliumhydrogenphosphat ($K_2HPO_4$ und/oder $KH_2PO_4$) enthalten. Eine Zerkleinerung der Candida-boidinii-Zellen ist nicht erforderlich. Diese erfindungsgemäße Ausführungsform beruht auf der Beobachtung, daß FDH nach dem Einfrieren der Zellen und ihrer Suspendierung in einem wässerigen phosphathaltigen

Medium zuerst und fast vollständig hinausdiffundiert, während ein großer Teil des Restproteins noch in den Zellen verbleibt.

Das bei einem Enzymaustritt von maximal etwa 95, 85 oder 75 % anfallende Medium kann auch Ausgangsmaterial für ein anderes FDH-Gewinnungsverfahren sein, das Medium muß also nicht der erfindungsgemäßen Phasenverteilung zugeführt werden.

Gemäß einer weiteren Ausführungsform des erfindungsgemäßen Verfahrens kann man die aufgeschlossene Zellsuspension erhitzen und danach wieder abkühlen. Dadurch werden einige Proteine koaguliert, die sich daraufhin leicht mit dem Zellmaterial abtrennen lassen. Außerdem werden hitzelabile Proteine, wie Dehydrogenasen oder Kinasen, desaktiviert, so daß sie keine Bindung mit dem Liganden eingehen können.

Vorzugsweise verwendet man ein wässeriges 2-Phasensystem mit

(a) Polyethylenglykol oder Polypropylenglykol und

(b) Rohdextran, Dextran, Methylcellulose oder Ficoll als Phasenbildner. Im übrigen ist der Fachmann mit der Wahl geeigneter 2-Phasensysteme vertraut, vgl. beispielsweise DE-C-26 39 129.7 und die darin angeführte Literatur.

Procion Red H-3B® (C.I.18159) hat die folgende Formel:

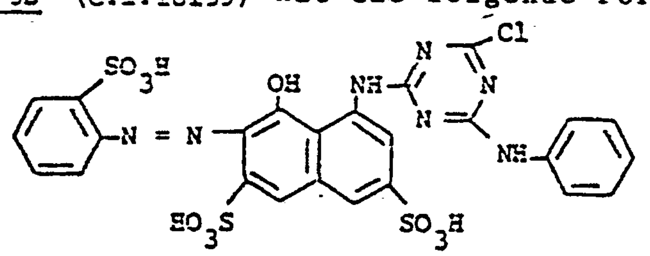

Zum Verknüpfen von Procion-red-HE3b® mit einem inerten wasserlöslichen Polymeren sei beispielsweise auf Analytical Biochemistry, 124 (1982) 117 bis 124, und die folgende Methode 1 verwiesen. Die an den beiden angegebenen Stellen zu findenden Angaben lassen sich auf andere inerte wasserlösliche Polymere übertragen.

Die Erfindung betrifft ferner ein Verfahren, bei dem man bei einem wässerigen 2-Phasensystem mit

(a) Polyethylenglycol oder Polypropylenglykol und

(b) Rohdextran, Dextran, Methylcellulose oder Ficoll als Phasenbildner und an Polyethylenglykol oder Polypropylenglykol gebundenem Procion-red-HE3b® als Ligand die Oberphase mit dem darin angereicherten Enzym von der Unterphase trennt und mit einem Salz, insbesondere Kaliumphosphat und/oder Kaliumhydrogenphosphat, versetzt und das Enzym in die Unterphase des resultierenden 2-Phasensystems überführt und gegebenenfalls (insbesondere durch Ultrafiltration) von Salzen und Phasenbildnern abtrennt.

Vorzugsweise wird die ligandenhaltige Oberphase wiederverwendet und vorzugsweise wird die Unterphase, in die das Enzym übergeführt worden ist, mit Polyethylenglykol oder Polypropylenglykol versetzt und die Oberphase des resultierenden 2-Phasensystems mit zuvor abgetrennter Oberphase vereinigt und danach wiederverwendet.

Es ist vorteilhaft, das in einem von Phasenbildnern und Salzen freien flüssigen Medium vorliegende Enzym in Gegenwart von Saccharose zu lyophilisieren, da das anfallende Produkt lagerbeständig ist.

Nachstehend wird die Erfindung näher erläutert.

Methode 1

Schritt 1: Chlorierung. Es wird von Monomethoxy-polyethylenglykol (MPEG) oder Polyethylenglykol (PEG) mit beispielsweise einem Molekulargewicht von 400, 1.500, 5.000, 6.000 oder 10.000 ausgegangen (im folgenden kurz PEG). Festes PEG oder MPEG wird über Nacht im Trockenschrank bei 70 °C geschmolzen. Das gegebenenfalls geschmolzene PEG oder MPEG wird anschließend unter Vakuum entwässert. Die Chlorierung erfolgt im Rotationsverdampfer mit einem 50-molaren Überschuß an Thionylchlorid bei 70 °C. Da wegen der Gefahr der HCl-Bildung absolut wasserfrei gearbeitet werden muß, wird das Reaktionsgefäß mit Stickstoff begast und auf den Kühler ein Trockenrohr aufgesetzt. Nach etwa 8 h ist die Reaktion beendet. Das überschüssige Thionylchlorid wird im Vakuum abgezogen.

Schritt 2: Aminierung. Das chlorierte PEG oder MPEG wird in einem großen Überschuß an wässerigem

Ammoniak (25 %) in einem Glasautoklaven (1,5 l) gelöst. Das Reaktionsgefäß sollte etwa zu 3/4 gefüllt sein; es wird dann luftdicht verschlossen. Danach wird die Aminierung im Ölbad bei 110 °Cetwa 30 h lang durchgeführt. Das Reaktionsprodukt wird im Rotationsverdampfer eingeengt, wobei überschüssiger Ammoniak entfernt wird.

Schritt 3: Kopplung. Auf 1 Mol $NH_2$ wird 1 Mol Procion-red-HE3b® gegeben. Danach wird der pH auf 11 eingestellt und die Reaktion bei 60 °C etwa 24 h lang durchgeführt. Das Reaktionsprodukt wird einer Gelfiltration (Sephadex-G50) unterworfen, um den freien Farbstoff abzutrennen, und danach dialysiert.

Beispiel 1

Schritt 1: Suspendierung. Es werden 10 kg gefrorene Hefe in einem Kaliumphosphat-Puffer über Nacht mit einem Propeller-Rührer suspendiert. Die Lösung enthält 40 % Zellmasse, 10 % Ammoniumformiat und 0,1 M Kaliumphosphat. Das Volumen beträgt 25 l. Am nächsten Morgen wird die Enzymaktivität gemessen.

Schritt 2: Hitzedenaturierung. Die Zellsuspension wird im Wasserbad (75 °C) erhitzt und unter Rühren bei 60 °C gehalten. Die Temperatur wird mit einem Thermometer im Reaktionsgefäß kontrolliert. Die Aufheizzeit beträgt etwa 15 min. Nach etwa 10 min bei 60 °C wird in Eiswasser auf Raumtemperatur abgekühlt.

Schritt 3: Affinitätsverteilung. Dazu werden eingewogen:

| | |
|---|---|
| 9 % PEG10000 (vermindert um den Anteil an PEG-red) (Polyethylenglykol mit einem mittleren Molekulargewicht von 10.000) | 4,356 kg |
| 43 mmol MPEG-red (Procion-red-HE3b® gebunden an Monomethoxy-polyethylenglykol mit einem mittleren Molekulargewicht von 5.000; als wässerige Lösung) | 1,240 kg |
| 1 % Rohdextran (10-proz. Lösung) | 0,500 kg |
| 25 l 40-proz. Zellsuspension (Dichte = 1,076 kg/l) | 26,900 kg |
| Rest Wasser auf 50 kg | |

Das System wird 30 min lang mit einem Blattrührer durchmischt. Dabei muß darauf geachtet werden, daß keine starke Schaumbildung erfolgt. Dann wird das System in einem Düsenseparator (4 Düsen mit einem Durchmesser von 0,4 mm) bei einer Durchflußgeschwindigkeit von 500 ml/min getrennt. Die Reinheit der Phasen beträgt 95 % für die Oberphase und 90 % für die Unterphase. Die gelartige Unterphase wird verworfen.

Schritt 4: Trennung Enzym/Ligand. Die Oberphase (38,57 l) wird mit 9 % Kaliumphosphat (Gewicht/Volumen; System: 10,5 % PEG10.000/9,9 % Kaliumphosphat) gemischt, nach Auflösung des Salzes noch 10 min lang gerührt und dann bei einer Durchflußgeschwindigkeit von 400 ml/min in einem Tellerseparator (Düsenlänge 14,5 mm) getrennt. Die Reinheit beider Phasen liegt bei 100 %. Die Oberphase wird für die Wiederverwendung bei -20 °C aufgehoben. Die Unterphase wird mit 1 % PEG10.000 vermischt und nach dessen Auflösung wieder mit 400 ml/min im Separator getrennt. Die Oberphase wird gemeinsam mit der Oberphase des ersten PEG/Salz-Systems gelagert.

Schritt 5: Ultrafiltration. Die Unterphase (27,3 l) wird in einem Kapillarmembran-System (Filtratdurchsatz 50 l/h) von Salzen und Polyethylenglykol befreit und auf ein Volumen von 5,28 l eingeengt. Danach wird in einer zweiten Kapillarmembran-Anlage weiter auf ein Endvolumen von 1 l auf konzentriert.

Schritt 6: Lyophilisation. Zu dem Retentat der Ultrafiltration werden 171 g Saccharose gegeben. Danach wird im Gefriertrockner lyophilisiert. Man erhält 243 g Produkt einer Aktivität von 0,23 U FDH/mg Lyophilisat.

Vergleichsbeispiel 1

Beispiel 1 wird mit der Ausnahme wiederholt, daß anstelle von 43 mmol MPEG-red eine wässerige Lösung mit einem Gehalt an 400 mmol MPEG-blue verwendet wird (Cibacron-blue-3G-A® gebunden an Monomethoxy-polyethylenglykol). Es wurde eine gegenüber Beispiel 1 geringere spez. Aktivität und Ausbeute erhalten.

**Patentansprüche**

1. Verfahren zur Reinigung und gegebenenfalls Gewinnung von Formiat-Dehydrogenase (FDH) aus Candida boidinii, bei dem man
   (a) das Enzym enthaltende Zellen aufschließt,
   (b) eine Phasenverteilung (Affinitätsextraktion) mit einem wässerigen 2-Phasensystem durchführt, in dessen einer Phase ein Triazinfarbstoff (Ligand) angereichert ist, der an ein inertes wasserlösliches Polymeres gebunden ist, und
   (c) das Enzym gegebenenfalls von der Phase abtrennt, in der es angereichert ist, und gegebenenfalls gewinnt,
   dadurch ***gekennzeichnet***, daß man bei der Stufe (b) die Phasenverteilung mit einem 2-Phasensystem durchführt, in dessen einer Phase Procion-red-HE3b® (Ligand) angereichert ist, der an ein inertes wasserlösliches Polymeres gebunden ist,
   - gegebenenfalls ohne vorherige Abtrennung der Zellen und Zelltrümmer und
   - gegebenenfalls ohne vorherige Abtrennung von Fremdprotein.

2. Verfahren nach Anspruch 1, dadurch ***gekennzeichnet***, daß man die Candida-boidinii-Zellen nach vorhergehendem Einfrieren in einem wässerigen phosphathaltigen Medium, insbesondere einem Medium mit einem Gehalt an Kaliumphosphat und/oder Kaliumhydrogenphosphat, suspendiert und in der Suspension bis zu einem Enzymaustritt von maximal etwa 90, 85 oder 75 % verweilen läßt und die erhaltene Suspension der Phasenverteilung unterwirft.

3. Verfahren nach Anspruch 2, dadurch ***gekennzeichnet***, daß man die Zellen nicht zerkleinert.

4. Verfahren nach Anspruch 2 oder 3, dadurch ***gekennzeichnet***, daß man die erhaltene aufgeschlossene Suspension erhitzt und danach wieder abkühlt, bevor man sie der Phasenverteilung unterwirft.

5. Verfahren nach einem der vorhergehenden Ansprüche, dadurch ***gekennzeichnet***, daß man ein wässeriges 2-Phasensystem mit
   (a) Polyethylenglykol oder Polypropylenglykol und
   (b) Rohdextran, Dextran, Methylcellulose oder Ficoll
   als Phasenbildner verwendet.

6. Verfahren nach einem der vorhergehenden Ansprüche, dadurch ***gekennzeichnet***, daß man als Liganden an Polyethylenglykol oder Polypropylenglykol gebundenes Procion-red-HE3b® verwendet.

7. Verfahren nach Anspruch 5 oder 6, dadurch ***gekennzeichnet***, daß man bei einem 2-Phasensystem gemäß Anspruch 5 oder 6 die Oberphase mit dem darin angereicherten Enzym von der Unterphase trennt und mit einem Salz, insbesondere Kaliumphosphat und/oder Kaliumhydrogenphosphat, versetzt und das Enzym in die Unterphase des resultierenden 2-Phasensystems überführt und gegebenenfalls (insbesondere durch Ultrafiltration) von Salzen und Phasenbildnern abtrennt.

8. Verfahren nach Anspruch 7, dadurch ***gekennzeichnet***, daß man die ligandenhaltige Oberphase wiederverwendet.

9. Verfahren nach Anspruch 7 oder 8, dadurch ***gekennzeichnet***, daß man die Unterphase, in die das Enzym übergeführt worden ist, mit Polyethylenglykol oder Polypropylenglykol versetzt und die Oberphase des resultierenden 2-Phasensystems mit der gemäß Anspruch 7 abgetrennten Oberphase vereinigt und danach wiederverwendet.

10. Verfahren nach einem der vorhergehenden Ansprüche, dadurch ***gekennzeichnet***, daß man das in

einem von Phasenbildnern und Salzen freien flüssigen Medium insbesondere nach Anspruch 7 vorliegende Enzym in Gegenwart von Saccharose lyophilisiert.

## Claims

1. Process for the purifying and, where appropriate, obtaining of formate dehydrogenase (FDH) from Candida boidinii, in which
   (a) the cells containing the enzyme are disrupted,
   (b) a phase partition (affinity extraction) is carried out with an aqueous two-phase system, in one phase of which a triazine dyestuff (ligand) which is bound to an inert water-soluble polymer is concentrated, and
   (c) the enzyme is, where appropriate, separated from the phase in which it is concentrated and, where appropriate, is isolated,
   characterised in that in stage (b) the phase partition is carried out with a 2-phase system, in one phase of which Procion red HE3b® (ligand) which is bound to an inert water-soluble polymer is concentrated,
   - where appropriate without previous removal of the cells and cell debris and
   - where appropriate without previous removal of foreign protein.

2. Process according to Claim 1, characterised in that the Candida boidinii cells are, after previous freezing, suspended in an agueous phosphate-containing medium, especially a medium containing potassium phosphate and/or potassium hydrogen phosphate, and left in the suspension until the enzyme emission is utmost about 90, 85 or 75 %, and the resulting suspension is subjected to the phase partition.

3. Process according to Claim 2, characterised in that the cells are not comminuted.

4. Process according to Claim 2 or 3, characterised in that the resulting disrupted suspension is heated and then cooled again before it is subjected to the phase partition.

5. Process according to any of the preceding claims, characterised in that an agueous 2-phase system containing
   (a) polyethylene glycol or polypropylene glycol and
   (b) crude dextran, dextran, methylcellulose or Ficoll as phase former is used.

6. Process according to any of the preceding claims, characterised in that Procion red HE3b® bound to polyethylene glycol or polypropylene glycol is used as ligand.

7. Process according to Claim 5 or 6, characterised in that in a 2-phase system according to Claim 5 or 6 the upper phase with the enzyme concentrated therein is separated from the lower phase and mixed with a salt, especially potassium phosphate and/or potassium hydrogen phosphate, and the enzyme is transferred into the lower phase of the resulting 2-phase system and, where appropriate, separated from salts and phase formers (especially by ultrafiltration).

8. Process according to Claim 7, characterised in that the ligand-containing upper phase is reused.

9. Process according to Claim 7 or 8, characterised in that the lower phase into which the enzyme has been transferred is mixed with polyethylene glycol or polypropylene glycol, and the upper phase of the resulting 2-phase system is combined with the upper phase removed as in Claim 7 and then reused.

10. Process according to any of the preceding claims, characterised in that the enzyme which is present in a liquid medium free of phase formers and salts, especially according to Claim 7, is lyophilised in the presence of sucrose.

## Revendications

1. Procédé de purification et éventuellement d'obtention de formiate-déshydrogénase (FDH) à partir de Candida boidinii, dans lequel
   (a) on casse les cellules contenant l'enzyme,

(b) on procède à un partage de phases (extraction d'affinité) dans un système aqueux biphasique, dont l'une des phases est enrichie en colorant triazinique (ligand), lequel est fixé à un polymère inerte soluble dans l'eau, et
(c) on sépare éventuellement l'enzyme de la phase dans laquelle elle s'est concentrée, et éventuellement on la récupère,

caractérisé en ce que, dans l'étape (b), on procède au partage de phases dans un système biphasique dont l'une des phases est enrichie en Procion-red-HE3b® (ligand), lié à un polymère inerte soluble dans l'eau,

- éventuellement sans séparation préalable des cellules et des débris cellulaires, et
- éventuellement sans séparation préalable des protéines étrangères.

**2.** Procédé selon la revendication 1, caractérisé en ce qu'on met en suspension les cellules de Candida biodinii, après congélation préalable, dans un milieu phosphaté aqueux, en particulier un milieu contenant du phosphate de potassium et/ou de l'hydrogénophosphate de potassium, où on les laisse séjourner jusqu'à la libération d'au maximum 90, 85 ou 75 % environ de la quantité d'enzyme, et on soumet la suspension obtenue au partage de phases.

**3.** Procédé selon la revendication 2, caractérisé en ce que les cellules ne sont pas broyées.

**4.** Procédé selon la revendication 2 ou 3, caractérisé en ce qu'on chauffe la suspension de cellules cassées obtenue, puis qu'on la refroidit de nouveau avant de la soumettre au partage de phases.

**5.** Procédé selon l'une des revendications précédentes, caractérisé en ce qu'on utilise un système biphasique aqueux comprenant, comme formateurs de phases
(a) du polyéthylène glycol ou du polypropylène glycol, et
(b) du dextrane brut, du dextrane, de la méthylcellulose ou du ficoll.

**6.** Procédé selon l'une des revendications précédentes, caractérisé en ce qu'on utilise comme ligand du Procion-red-HE3b® lié à du polyéthylène glycol ou à du polypropylène glycol.

**7.** Procédé selon la revendication 5 ou 6, caractérisé en ce que, en présence d'un système biphasique selon la revendication 5 ou 6, on sépare de la phase inférieure la phase supérieure avec l'enzyme qui s'est concentrée et on lui ajoute un sel, en particulier du phosphate de potassium et/ou de l'hydrogénophosphate de potassium, et on fait passer l'enzyme dans la phase inférieure du système biphasique obtenu, et éventuellement on la sépare (notamment par ultra-filtration) des sels et des formateurs de phases.

**8.** Procédé selon la revendication 7, caractérisé en ce qu'on réutilise la phase supérieure contenant le ligand.

**9.** Procédé selon la revendication 7 ou 8, caractérisé en ce qu'à la phase inférieure dans laquelle est passée l'enzyme, on ajoute du polyéthylène glycol ou du polypropylène glycol et on réunit la phase supérieure du système biphasique obtenu et la phase supérieure séparée selon la revendication 7, puis on la réutilise.

**10.** Procédé selon l'une des revendications précédentes, caractérisé en ce qu'on lyophilise l'enzyme présente dans un milieu liquide exempt de formateurs de phases et de sels, en particulier le milieu obtenu à la revendication 7, en présence de saccharose.